# EUROPEAN PATENT APPLICATION

(11) **EP 1 905 761 A1**
(43) Date of publication of application: **02.04.2008**
(21) Application number: 07018655.6
(22) Date of filing: 22.09.2007
(51) Int. Cl.: C07D 213/74

(54) **An improved process for the preparation of 4-[2-(methyl-2-pyridinylamino) ethoxy] benzaldehyde, an intermediate for the preparation of rosiglitazone**

(30) Priority: 22.09.2006 IN MU15172006
(71) Applicant: Cadila Pharmaceuticals Limited, Bhat, Ahmedabad 382 210 (IN)
(72) Inventor: Modi, Indravadan Ambalai, Bhat Ahmedabad - 382 210 (IN); Shukla, Manish Chandrakant, Dr., Bhat Ahmedabad - 382 210 (IN); Sondagar, Keval Rameshkumar, Bhat Ahmedabad - 382 210 (IN); Joshi, Atul Chhotalal, Bhat Ahmedabad - 382 210 (IN); Ponnaiah, Ravi, Dr., Bhat Ahmedabad - 382 210 (IN); Khamar, Bakulesh Mefatlal, Dr., Bhat Ahmedabad - 382 210 (IN)
(74) Representative: Towler, Philip Dean

(57) **Abstract**

The invention relates to an improved process for the preparation of 4-[2-(methyl-2-pyridinylamino)ethoxy]-benzaldehyde via 4-[2-(methyl-2-pyridinylamino).ethoxy]-benzonitrile.

The process of preparation of 4-[2-(methyl-2-pyridinylamino)ethoxy]-benzaldehyde comprises:
(a) Reacting 2[methyl(pyridine-2-yl)amino]ethanol with 4-halobenzonitrile using a base in a solvent, to give 4-(2-[methyl(pyridine-2-yl)amino]ethoxy)benzonitrile,
(b) reacting a solution of 4-(2-[methyl(pyridine-2-yl)amino]ethoxy)benzonitrile in formic acid with Raney nickel in water ,to give 4-[2-(methyl-2-pyridinylamino)ethoxy]-benzaldehyde.

## Description

### FIELD OF INVENTION :

The present invention relates to an improved process for the preparation of 4-[2-(methyl-2-pyridinylamino)ethoxy]-benzaldehyde via 4-[2-(methyl-2-pyridinylamino)ethoxy]-benzonitrile

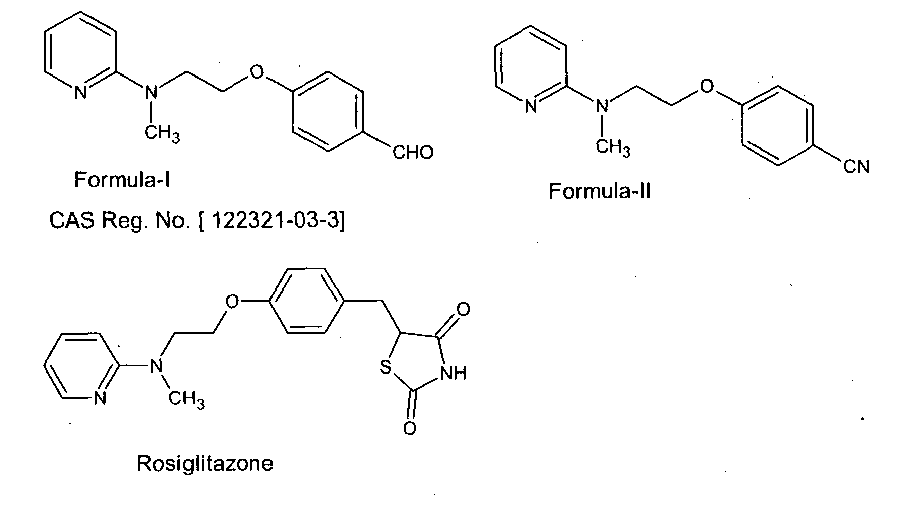

### BACKGROUND OF INVENTION

European patent application 0306228, describes the reaction of 2-(N-methyl-N-(2-pyridyl)amino)ethanol with 4-fluorobenzaldehyde in the presence of dimethyl formamide as solvent and sodium hydride as a base.

Cantello et. Al. (J. Med. Chem. 1994,37,3977-85 prepared rosiglitazone and reported yield of 48 % for the reaction of 2-(N-methyl-N-(2-pyridyl) amino)ethanol with 4-fluorobenzaldehyde at room temperature in the presence of dimethyl formamide as solvent and sodium hydride as base for the synthesis of 4-[2-(N-methyl-N-(2-pyridyl)amino)ethoxy]benzaldehyde.
Further Cantello et. al. ( Bioorg & Med. Chem.. Letters vol 45,p.1181-84 (1994) have reported yield of 72 % when the same reaction is carried out at 80°C.

WO 2002/051823 discloses the reaction of 2-(N-methyl-N-(2-pyridyl)amino)ethanol with 4-fluorobenzaldehyde and potassium tert. butoxide in N,N-dimethyl formamide as solvent .The reaction mass was quenched in water and extracted in ethyl acetate with further work up to give 9-[2-(N-methyl-N-(2-pyridyl)amino)ethoxy]benzaldehyde with 88 % yield.

Li, Jiaming; Li, Feng; Cha, Dajun describe in Zhongguo Yaowu Huaxue Zazhi (2001), 11(5), 291-292, 294 , describe the synthesis of rosiglitazone wherein 2-chloropyridine is reacted with 2-methylaminoethanol at 120°C for 18 h under bubbling N2, and reacted with 4-fluorobenzaldehyde in DMF in the presence of KOH and TEBA at 120°C for 10 h under bubbling N2 to obtain 4-[2-(N-methyl-2-pyridylamino)ethoxy]benzaldehyde.

WO 200100044240 describes the preparation of 4-[2-(N-methyl-2-pyridylamino)ethoxy]benzaldehyde wherein pot. tert. butoxide in DMF was added to 2-(N-methyl-N-(2-pyridyl)amino)ethanol in DMF, followed by addition of 4-fluorobenzaldehyde in DMF, further stirred under nitrogen for 4 hours at 80°C and worked up to give 4-[2-(N-methyl-2-pyridylamino)ethoxy]benzaldehyde in 77 % yield which was used directly in the next step.

The publication Huaxue Shiji (2000), 22(6), 372 describes an improved method for the synthesis of 4-[2-(methyl-2-pyridinylamino)ethoxy]benzaldehyde. Reaction of 2-chloropyridine with 2-methylaminoethanol at 120°C for 18 h gave 73.3% 2-(N-methyl-N-(2-pyridyl)amino)ethanol , condensation of which with p-fluorobenzaldehyde in DMF in the presence of KOH and triethylbenzylammonium chloride at 120°C for 10 hours gave 76.1% 4-[2-(methyl-2-pyridinylamino)ethoxy]benzaldehyde
US 5002953 describes preparation of 4-[2-(methyl-2-pyridinylamino)ethoxy]benzaldehyde [in preparation-33 , by an analogous process described in preparation 22] as follows:
Sodium hydride ( 60 % dispersion in oil) was added to a stirred solution of 2(N-methyl-N-(2-pyridyl)amino)ethanol in DMF under an atmosphere of nitrogen. After the vigorous reaction was subsided, 4-fluorobenzaldehyde was added and the reaction mixture was stirred at 80°C for 16 hours , after cooling, the reaction mixture was added to water and extracted with diethyl ether, organic extracts were washed with brine, dried over anhydrous Magnesium sulfate ,filtered and evaporated to dryness. The product was column chromatographed over silica-gel using 1% methanol in dichloromethane.

WO2007/017095 discloses a process for the preparation of a compound of formula-(ii) 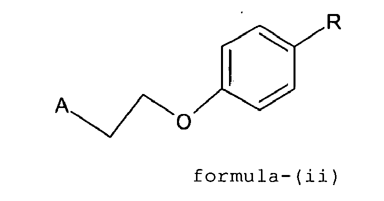 wherein
A is selected from (a) aryl group,(b) a phenyl group optionally substituted by one or two substituents each selected from nitro,halo,C1-C4 alkyl,C1-C4 alkoxy and hydroxyl, (c ) al- or 2-naphthyl group,(d) pyridyl optionally substituted by C1-C4 alkyl group, 5- or 6- membered unsaturated heterocyclic ring containing from one to three heteroatome selected from nitrogen, oxygen or sulfur, 5-ethyl-2-pyridinyl or N-methyl-N-(2-pyridyl)amino radical, R is aldehyde, cyano or nitro group, which comprises:
Reacting a compound of formula (iii) 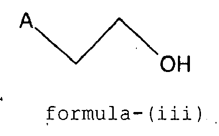
wherein A is as defined above, with a compound of formula (iv) 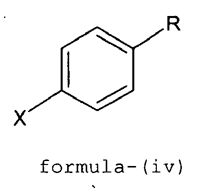 wherein X is chlorine, bromine, fluorine and R is as defined above, in a mixture of a non-polar water immiscible organic solvent and water with an alkali metal hydroxide or an alkali metal carbonate as a base in the presence of a phase transfer catalyst.

The prior art processes give low yields, involve multi-step extractive work up procedures ,give impure products and degradation products, involve chromatographic purification, and require relatively long reaction times.

It is a long-felt need of the industry to provide a process which does not have these disadvantages.

### SUMMARY OF THE INVENTION

The main object of the invention is to provide a high yielding process for the preparation of 4-[2-(methyl-2-pyridinylamino)ethoxy]-benzaldehyde, in substantially pure form without elaborate work up procedures.

Yet another object of the present invention is to provide a process for the preparation of formula-I with relatively shorter reaction times

Yet another object of the present invention is to provide a process for the preparation of formula-I which does not give impure products and degradation products at the etherification step.

Yet another object of the present invention is to develop a process for the preparation of formula-I which does not involve chromatographic purification technique.

It is yet another object of the present invention to prepare a compound of formula-II without using a phase transfer catalyst.

Yet another object of the present invention is to provide a process for the preparation of formula-I with a purity of about 98 % to 99 %.

Yet another object of the present invention is to provide a process for the preparation of formula-II with a purity > 99 % by HPLC

### DETAILED DESCRIPTION

The present invention is described in scheme-A: 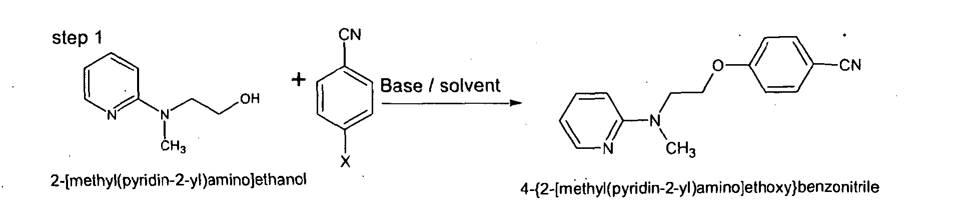 X=F,Cl,Br

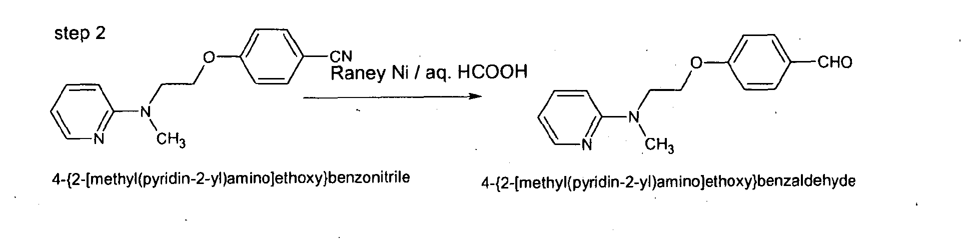

### Step-1

2-[methyl(pyridin2-yl)amino]ethanol is reacted with 4-halobenzonitrile, using a base in a solvent to give 4-{2-[methyl(pyridin-2-yl)amino]ethoxy]benzonitrile In 4-halobenzonitrile the X= Cl, Br, F ;
The solvent used for this step is selected from :
substituted amide solvents like DMF , N,N-dimethyl acetamide, N-methyl pyrrolidone, 1,1,3,3, tetramethyl urea, 1,3-dimethyl imidazolidin2-one and their like, or mixtures thereof; aromatic solvents like Benzene, toluene, Xylene, ethylbenzene or mixtures thereof; ethers like THF ,2-Methyl THF, di-n-propyl ether, di-n-butyl ether, monoglyme, diglyme 1,4- dioxane; DMSO, sulfolane,and their like, or mixtures thereof;

The preferred solvents are DMF and N,N-dimethyl acetamide , or mixtures thereof.

The base used for this condensation reaction is selected from :
hydrides such as NaH, LiH, CaH2 ; alkoxides of C1 to C5 alcohols such as tert. butoxides of Na and K, Li ; isopropoxides of Na, K; the preferred bases among this group being sodium hydride, potassium tert. butoxide and sodium tert. butoxide ; the more preferred base being sodium hydride; alkali metal carbonates such as Na2CO3, K2CO3 Li2CO3, Cs2CO3 , the preferred base of this group is K2CO3 in polar aprotic solvent such as DMF or DMSO. Alkali metal hydroxides such as NaOH, KOH, LiOH, Ca(OH)2, Ba(OH)2 , the more preferred among these are NaOH or KOH with DMSO or sulfolane; tertiary amine bases such as DBU,DBN, TMEDA, and their like;

The reaction is most preferably carried out at 30-35°C using sodium hydride in DMF.

In a preferred embodiment, a solution of 2-(N-methyl-N-(2-pyridyl)amino)ethanol) in DMF is added to sodium hydride in DMF at 30-35° C over about 30 minutes, stirred for about one hour. A solution of 4-chlorobenzonitrile is added over about one hour.The reaction mass is stirred for about four hours. The progress of this reaction is determined by TLC. The reaction product separated by aqueous quenching of reaction mass.

### Step-2

4-{2-[methyl(pyridin-2-yl)amino]ethoxy]benzonitrile is reacted with Raney Nickel and aqueous formic acid to give 4-[2-(methyl-2-pyridinylamino)ethoxy]-benzaldehyde.

The reference method to effect this conversion is as given in organic Syntheses collective volume-6 page 631.

Raney nickel is taken up in water and to it is added a solution of nitrile dissolved in formic acid at 30-35°C; The initial quantity of water is taken in such a way that the concentration of formic acid becomes ~75 % v/v.

The reaction mass is stirred at 95-100°C and maintained at this temperature for 4 hours. The reaction mass is cooled to 30-35°C and filtered thru hyflo bed. The filtrate is cooled to 5-10°C and pH was adjusted to 4.0 to 6.0 preferably at 4.7 to 5 using aqueous ammonia [* Other bases such as NaOH, KOH , Na2CO3 , K2CO3, CsCO3 can also be used]. Solid material is separated , filtered and characterized as 4-[2-(methyl-2-pyridinylamino)ethoxy]-benzaldehyde.

The advantage of the present invention is that 4-[2-(methyl-2-pyridinylamino)ethoxy]-benzaldehyde is isolated with a purity of about 99 %.

Another advantage of the present invention is that 4-[2-(methyl-2-pyridinylamino)ethoxy]-benzonitrile is isolated with a purity of > 99 %.

The present invention is illustrated with following examples which do not limit the scope of this invention.

### Example-1

Preparation of 4-[2-(methyl-2-pyridinylamino)ethoxy]-benzonitrile N,N-dimethylformamide (1500 ml) was charged, under nitrogen atmosphere, in round bottom flask equipped with mechanical stirrer, addition funnel , condenser and guard tube. Sodium hydride ( ~55-60 % in paraffin oil-174.48 gm) was added to it at 30 to 35°C. The reaction mass was stirred for 15 minutes and to it a solution of 2-(N-methyl-N-(2-pyridyl)amino)ethanol) (500 gm) in 1000 ml of N,N-dimethyl formamide was added at 30 to 35°C over about 30 minutes. The reaction mass was stirred for one hour at 30 to 35°C. A solution of 4-chlorobenzonitrile (451.52 gm ) in 1000 ml of N,N-dimethyl formamide was added to it at 30 to 35°C over one hour. The reaction mass was stirred for about four hours at 30 to 35°C. The progress of the reaction was monitored by TLC. The reaction mass was then dumped into 10.5 L of chilled water. The reaction mass was stirred for 30 minutes. The reaction mass was extracted with toluene (3500ml x 2) and combined toluene layer was filtered through hyflo bed and washed with water (10.5L x 2 ) and dried over anhydrous sodium sulfate. The solvent was stripped off with cyclohexane under vacuum to give oily mass. Cyclohexane (1500 ml) was added to this and stirred for 3-4 hours. The solid was filtered and dried.
Weight = 650 gms
Yield = 78.20 %
HPLC purity=99.20 %
Mass= 254.1(M+1)
Proton NMR :
δ values : 3.125(3H),3.983-4.021 (2H),4.215-4.253 (2H),6.494-6.594(2H) 6.930-6.960 (2H),7.434-7.464 (1H),7.529-7.559 (2H), 8.138-8.163 (1H).

### Example-2

Preparation of 4-[2-(methyl-2-pyridinylamino)ethoxy]-benzaldehyde from 4-[2-(methyl-2-pyridinylamino)ethoxy]-benzonitrile.

Raney Nickel (300 gm) in 212 ml of water were charged in 5 lit Round bottom flask equipped with stirrer, condenser, dropping funnel, thermometer. The reaction mass was stirred for 10 minutes. A solution of 4 - [2 - pyridinylamino)ethoxy] - benzonitrile (600 gm) dissolved in 1588 ml of 85 % formic acid were added to it at 30 - 35°C. After the addition was over, the reaction mass was stirred up to 100 - 105°C and maintained at 100 - 105°C for four hours. The reaction mass was cooled to 30 - 35°C and filtered through hyflo bed. The filtrate was cooled to 5 - 10°C, pH of the filtrate was adjusted to 4.7 to 5 using 25 % aqueous ammonia. The solid separated was filtered and washed with water. The product was characterized as 4 - [ 2 - (methyl - 2 - pyridinylamino)ethoxy] - benzaldehyde.

| | |
|---|---|
| Weight = | 490 gm |
| % Yield = | 82.00 % |
| HPLC Purity = | 99.05 % |
| Mass = | 257.2 (M+1) |

Proton NMR :
δ values : 3.134(3H),3.994-4.032 (2H),4.251-4.289 (2H),6.497-6.587(2H) , 6.981-7.011 (2H),7.435-7.487 (1H),7.776-7.822 (2H), 8.147-8.168 (1H),9.855 (1H).

## Claims

1. A process of preparation of 4-[2-(methyl-2-pyridinylamino)ethoxy]-benzaldehyde comprising:
(a) Reacting 2[methyl(pyridine-2-yl)amino]ethanol with 4-halobenzonitrile using a base in a solvent, to give 4-(2-[methyl(pyridine-2-yl)amino]ethoxy)benzonitrile,
(b) reacting a solution of 4-(2-[methyl(pyridine-2-yl)amino]ethoxy)benzonitrile in formic acid with Raney nickel in water ,to give 4-[2-(methyl-2-pyridinylamino)ethoxy]-benzaldehyde.

2. A process of preparation of 9-[2-(methyl-2-pyridinylamino)ethoxy]-benzaldehyde as claimed in claim-1 wherein halogen in 4-halobenzonitrile in step-a is F,C1,Br,I preferably F or Cl.

3. A process of preparation of 4-[2-(methyl-2-pyridinylamino)ethoxy]-benzaldehyde as claimed in claim-1 wherein solvent in step-a is selected from polar aprotic solvents such as N,N-dimethyl formamide, N,N-dimethyl acetamide, dimethyl sulfoxide, N,N,N',N'-tetramethyl urea, sulfolane, N,N'dialkyl cyclic urea, N-alkyl 2-pyrrolidone; ethers such as THF ,2-Methyl THF, di-n-propyl ether, di-n-butyl ether, monoglyme, diglyme 1,4- dioxane ; aromatic hydrocarbon solvents such as toluene, xylene.

4. A process of preparation of 4-[2-(methyl-2-pyridinylamino)ethoxy]-benzaldehyde as claimed in claim-1 wherein the preferred solvent in step-a is DMF.

5. A process of preparation of 4-[2-(methyl-2-pyridinylamino)ethoxy]-benzaldehyde as claimed in claim-1 wherein the base in step-a is selected from hydrides and alkoxides of of alkali metals and alkaline earth metals , alkali metal hydroxides and carbonates.

6. A process of preparation of 4-[2-(methyl-2-pyridinylamino)ethoxy]-benzaldehyde as claimed in claim-1 wherein the preferred base in step-a is sodium hydride.

7. A process of preparation of 4-[2-(methyl-2-pyridinylamino)ethoxy]-benzaldehyde as claimed in claim-1 wherein the reaction in step-a is carried out at 0°C to 100°C , preferably at 15-75°c more preferably at 30-35°C.

8. A process of preparation of 4-[2-(methyl-2-pyridinylamino)ethoxy]-benzaldehyde as claimed in claim-1 wherein in step-b the reaction is carried out at about 70 to 105°C, preferably at 100-105°C.

9. A process of preparation of 4-[2-(methyl-2-pyridinylamino)ethoxy]-benzaldehyde as claimed in claim-1 wherein in step-b after the reaction is over, the product of step-b is separated by adjusting the pH to 4.0 to 6.0 preferably at about 4.7 to 5 using a base such as hydroxides and carbonates, bicarbonates of alkali metals, aqueous ammonia.

10. A process of preparation of 4-[2-(methyl-2-pyridinylamino)ethoxy]-benzaldehyde as claimed in claim-1 wherein in step-b, claim-9, wherein the pH is adjusted using aqueous ammonia.
